# EUROPEAN PATENT APPLICATION

(11) **EP 3 598 458 A1**
(43) Date of publication of application: **22.01.2020**
(21) Application number: 19186388.5
(22) Date of filing: 15.07.2019
(51) Int. Cl.: G16H 20/40, A61B 17/00, G16H 40/63

(54) **MINIMALLY INVASIVE PROCEDURE ANALYSIS AND REVIEW SYSTEM AND METHOD**

(30) Priority: 18.07.2018 US 201816039129
(71) Applicant: General Electric Company, Schenectady, NY 12345 (US)
(72) Inventor: WARNER, Adrian F., Wauwatosa, WI 53226 (US); SCHNEIDEWEND, Daniel R., Wauwatosa, WI 53226 (US); MABINI, Daniel R., Wauwatosa, WI 53226 (US); NEKICH, Nicholas, Wauwatosa, WI 53226 (US)
(74) Representative: Fennell, Gareth Charles

(57) **Abstract**

A minimally invasive procedure analysis and review system includes a display device, a user input device, a computing system communicatively connected to the display device and the user input device, and a study analysis module executable on a processor. The study analysis module is configured to receive a running tally of events during the minimally invasive procedure, wherein each event includes an event time and an event type. An event is selected from the running tally of events, and at least two relevant datasets are determined based on the event type of the selected event. A relevant time portion of each relevant dataset is identified based on the event time of the selected event, and the relevant time portions of each of the at least two relevant datasets is displayed on the display device.

## Description

### BACKGROUND

The present disclosure generally relates to systems and methods for analyzing data gathered during a minimally invasive procedure, such as an invasive cardiology or electrophysiology procedure, and more specifically relates to an analysis and review system providing time synchronized display of multiple datasets, including, but not limited to, data collected by an electrophysiology system and image data captured by one or more imaging devices. Minimally invasive procedures, such as those performed in a cardiac catheterization laboratory, involve the collection of data relating to multiple different modalities, including measurement data from sensors in catheters, from physiological recording devices or modalities (e.g., ECG surface electrodes, physiological electrodes in catheters, invasive and noninvasive blood pressure monitors, respiration monitors, electroencephalographs, SpO₂ monitors, etc.) and from imaging devices (e.g., ultrasound, x-ray, computed tomography, magnetic resonance, nuclear (PET), 3D mapping, or optical CT imaging devices). Many different minimally invasive procedures may be performed, such as in the catheterization laboratory, utilizing some or all of the foregoing devices and systems, including angiography studies, electrophysiology studies, stent placements, and cardiac ablation, to name a few.

The x-ray images are acquired using cardiovascular x-ray imaging equipment. The resulting images are stored in digital form as DICOM (Digital Imaging and Communications in Medicine) images and stored and viewed electronically. These digital images are available for review and analysis at a physician review workstation.

During minimally invasive procedures, the patient also undergoes physiological recording modalities using a hemodynamic recording system. The hemodynamic recording system hooks up to a patient via externally placed leads that monitor the electrical impulses from the heart and records the heart's electrical activity in the form of a waveform. This record, called an electrocardiogram (ECG), is analyzed by well-known software that measures the heart's rhythms and electrical impulses, allowing the physician to detect heart irregularities, disease and damage. The ECG data, including waveforms and results of analysis, is typically stored in a computer database.

Additional data sources include electrodes on catheters inserted into the patient to measure electrical activity from within the heart, as well as a number of other types of physical modality sensors on catheters, including pressure sensor, temperature sensors, and current sensors.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one embodiment, a minimally invasive procedure analysis and review system includes a display device, a user input device, a computing system communicatively connected to the display device and the user input device, and a study analysis module executable on a processor. The study analysis module is configured to receive a running tally of events during the minimally invasive procedure, wherein each event includes an event time and an event type. An event is selected from the running tally of events, and at least two relevant datasets are determined based on the event type of the selected event. A relevant time portion of each relevant dataset is identified based on the event time of the selected event, and the relevant time portions of each of the at least two relevant datasets is displayed on the display device.

One embodiment of a method of operating a computing system to facilitate analysis and review of datasets collected during a minimally invasive procedure on a patient, wherein a computing system provides a graphical user interface on a display device and receives input from a user input device, includes receiving a running tally of events during a minimally invasive procedure, wherein each event includes an event time and an event type. A selected event is received from the running tally of events, and at least two relevant datasets are determined based on the event type of the selected even. A relevant time portion of each relevant dataset is then identified based on the event time of the selected event, and the relevant time portions of each of the at least two relevant datasets is displayed on a display device.

Another embodiment of a method of operating a computing system to facilitate analysis and review of datasets collected during a minimally invasive procedure includes providing multiple datasets, one for each of multiple modalities collected during the minimally invasive procedure, wherein all of the datasets are time synchronized to a reference clock, and then identifying at least two relevant datasets for display out of the multiple datasets. A selected time period is identified according to the reference clock, and a relevant time portion of each relevant dataset is identified based on the selected time period. The relevant time portions of each of the at least two relevant datasets is then displayed on a display device. A user input is received to adjust the selected time period, and an adjusted selected time period is identified based on the user input and according to the reference clock. An updated relevant time portion of each relevant dataset is identified to include data occurring during the adjusted selected time period, and the display for each of the at least two relevant datasets is adjusted to display the updated relevant time portions of each of the at least two relevant datasets on the display device.

Various other features, objects, and advantages of the invention will be made apparent from the following description taken together with the drawings.

### BRIEF DESCRIPTION OF THE DRAWINGS

The present disclosure is described with reference to the following Figures.
Figure 1 is a schematic block diagram depicting an exemplary minimally invasive procedure and analysis review system according to one embodiment of the present disclosure.
Figure 2 schematically depicts a computing system configured to receive certain inputs and provide certain outputs in accordance with an embodiment of the present disclosure.
Figure 3 is an exemplary time-correlated display in accordance with an exemplary embodiment of the present disclosure.
Figures 4-6 are flow charts depicting exemplary methods, or portions thereof, for operating a computing system to facilitate analysis and review of datasets collected during a minimally invasive procedure illustrating exemplary embodiments of the present disclosure.

### DETAILED DESCRIPTION

The present inventors have recognized that current minimally invasive procedures involve a multitude of devices each providing data that needs to be accounted for during various portions of the procedure. Accordingly, the inventors have recognized that clinicians performing or involved in minimally invasive procedures suffer from information overload and fatigue, especially in electrophysiology studies. Accordingly the inventors have recognized that systems and methods are needed for associating disparate data sources, assessing differing technical standards, and monitoring the operation of multiple data collection systems. Moreover, the inventors have recognized that an analysis and review system is needed that correlates and presents relevant sources of data in a time-correlated manner and in association with thorough and domain-aware event marking and analysis.

The inventors have further recognized that currently available systems present the various datasets independently. For example, images collected during the minimally invasive procedure are presented separately from physiological signal data, which are presented separately still from catheter data, such as from temperature sensors, pressure sensors, or current sensors on a catheter. Thus, using current systems to observe, review, and make determinations based on the multitude of available data is a laborious process and can be time intensive. Often, clinicians have limited time during procedures to assess datasets and make decisions. Accordingly, the inventors have further recognized that, given the data overload and difficulty of navigating the various data sets, clinicians are too often unable or too overloaded to sufficiently review the data in order to make an informed decision during a procedure.

In view of the forgoing problems and challenges with current systems recognized by the inventors, they developed the disclosed analysis and review system for analyzing multiple different types of datasets from different modalities collected during a minimally invasive procedure-e.g., from any of various sensors on catheters, from various physiological recording devices used to gather physiological information about the patient, from the patient's medical history set forth in their medical record, and/or data entered in by a clinician during a procedure (such as lab results or clinician observations about the patient). Each dataset includes data values-e.g., physiological measurements, image data, sensed pressure or temperature values, lab test values-and parameter values. Parameter values describe information about the dataset, such identifying the device that collected the data, physical conditions or settings of the collection device (e.g., mode settings, position of the c-arm or other x-ray device, x-ray intensity). Parameter values also include a time parameter indicating relevant time, such as a time value for each data value in a physiological dataset.

The procedural analysis and review system selects one or more relevant datasets in a time-synchronized and correlated way. Relevant datasets are datasets from the multiple available datasets that are identified as relevant based on the problem domain and/or based on clinician input. For example, relevant datasets may be identified based on an event selection by a clinician from the running tally of events in the procedure. The relevancy determination is made based on one or more of the parameter values for that dataset, such as by identifying the parameter values that are correlated to the event type of the selected event.

The relevant datasets may be identified based on a user-selected dataset or modality, such as selecting and controlling a time period of a particular dataset of the user-selected dataset. In other embodiments, the relevant datasets may be identified based on a user-selected event. For example, the relevant datasets may be identified based on a user-selected event from a running tally of events identified during the procedure. In either embodiment of relevant dataset identification, the system adjusts and updates review windows of all relevant datasets together, such that an adjustment to the time period in one of the relevant dataset windows is reflected in the displays of all of the other relevant datasets as well so that all of the modality windows correlate with the selected time period.

For example, the disclosed analysis and review system may analyze and intelligently present data collected by an electrophysiology system and by one or more imaging devices, such as x-ray imaging devices and/or ultrasound imaging devices, during the minimally invasive procedure. The analysis and review system receives and analyzes time-sequenced information and correlates the data in time. The system further catalogs and recognizes events, such as clinician marked events, programed, or procedure, events, or events detected based on the data itself (such as when one or more of the data values for one or more datasets exceeds a relevant threshold). Thereby, the disclosed system and method provides a means for efficiently observing and reviewing multiple data sets over a procedure period and for identifying and reviewing the most important and relevant data in order to make informed determinations during the procedure. Similarly, the disclosed system and method provide clinicians the ability to review time-synchronized images and data, and to navigate between physiological data and information collected throughout the procedure, to provide a comprehensive review of all the datasets within one uniform and easy-to-navigate user interface environment.

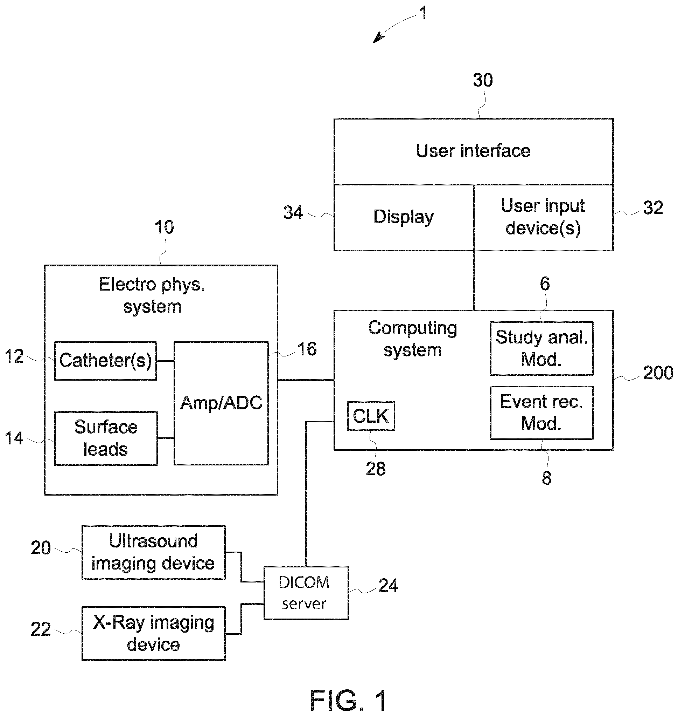

Figure 1 depicts an exemplary minimally invasive procedure analysis and review system 1. The system 1 comprises a computing system 200 controlling a user interface 30. The computing system 200 receives datasets from an electrophysiology system 10 comprising or connected to one or more catheters 12 and surface leads 14 in order to conduct an electrophysiology study, activation mapping, ablation intervention, or hemodynamic study, FFR analysis, stent placement, etc. The electrophysiology system includes an amplifier and analog-to-digital converter (ADC) 16 to digitize the various signals received from the one or more catheters 12 and surface leads 14, and such digitized signals are provided to the computing system 200. The computing system 200 further receives image data captured by one more imaging devices, exemplified as an ultrasound imaging device 20 and an x-ray imaging device 22. For example, the ultrasound imaging device 20 may be an ultrasound imaging catheter, such as an esophageal catheter used to take ultrasound images of the heart during a minimally invasive procedure. The x-ray imaging device 22 may include any of various available x-ray imaging devices that are commonly used during minimally invasive procedures, such as a c-arm system. The schematic diagram at Figure 1 represents that image data captured by the imaging devices 20, 22 is accessible by the computing system 200 through a DICOM server 24. The DICOM server 24 manages or incorporates a database of images stored as DICOM objects, which it accesses to retrieve image data when called to do so by the computing system 200. Given that the amount of image data acquired during a minimally invasive procedure can be quite substantial, requiring large amounts of memory and processing power, the image data from the imaging devices 20, 22 may be provided to a dedicated image server, such as a DICOM server dedicated to processing and storing the image data. Thus, the computing system 200 may retrieve stored image data from the DICOM server 24, which are then displayed on the display device 34 of the user interface 30. In certain embodiments, the data collected by the electrophysiology system 10 may also be provided to the DICOM server 24 and encapsulated and stored as DICOM objects. However, other arrangements are known for providing the image data from the imaging devices 20, 22 to the computing system 200, and all such alternative arrangements are within the scope of the disclosure.

Each of the multiple datasets provided to the computing system 200 are time-stamped in a way that the various datasets can be time-correlated. In one embodiment, all of the datasets is time synchronized to a reference clock, and the running tally of events identified during the procedure is also organized according to the same reference clock so that all data and events can be correlated accordingly by the system 1. In various embodiments, the reference clock 28 may be provided in the computing system 200, as in the depicted embodiment, or may be provided and/or associated with the DICOM server 24. In one embodiment, each frame of image data acquired by an imaging device 20, 22 is time-stamped with local time according to a clock located in or associated with the respective imaging device 20, 22. The time stamp may be embedded or encapsulated in the image data file, or object, such as inserted into a predetermined field in a header in the DICOM object. Similarly, the data collected during the procedure by the electrophysiology system 10 is also time-stamped according to a local clock in the electrophysiology system 10, such as a clock associated with the ADC 16. Each local clock located in each respective imaging device 20, 22 and the electrophysiology and vascular recording system 10 is then synchronized to a reference clock 28, which in various embodiments may be provided in the DICOM server 24 or in the computing system 200. For example, each imaging device 20, 22 and the electrophysiology and vascular recording system 10 may be configured to determine respective offsets of its local clock relative to the reference clock 28. Alternatively, the computing system 200 may be configured to determine and monitor the respective offsets for the local clocks and each of the associated systems and devices 10, 20, 22. The offsets may be stored in association with each data file or object, such as in a designated offset field in the header in the DICOM object or other file-type object. An exemplary system for synchronization of cardiovascular images and physiological or hemodynamic recording data is described at U.S. Patent No. 7,280,864, which is hereby incorporated by reference in its entirety.

In still other embodiments, the various datasets may be correlated by other means, such as according to one of the local clocks. Thus, for example, offsets may be determined between the clock associated with the electrophysiology and vascular recording system 10 and each of the local clocks associated with the imaging devices 20, 22. For example, each of the local clocks in the electrophysiology and vascular recording system 10 and the imaging devices 20, 22 may be adjusted in real-time using network time protocol (NTP) time synchronization or by some other time synchronization protocol, which may be to a separate designated reference clock 28, or to a respective one of the local clocks.

The computing system 200 comprises a study analysis module 6, which is software module executable to identify one or more datasets to be displayed and to display those datasets in a time-correlated manner. In certain embodiments, the computing system 200 may also comprise one or more event recognition modules 8 configured to assess the datasets, such as from the catheters 12 and/or surface leads 14, to detect events. For example, the event recognition module 8 may be executable to assess physiological signal data and catheter data from the electrophysiology and vascular recording system 10 and identify a threshold triggered event when the physiological signal data for one or more physiological modalities exceeds a relevant physiological threshold, or when the catheter data for one or more catheter measurement modalities exceeds a relevant physical measurement (e.g., pressure, temperature, etc.) threshold set for a respective catheter modality. One example in EP might be to monitor the invasive blood pressure channel, which is often used with a transseptal needle, to indicate when the septum has been breached. This is done to enable a sheath to be inserted to allow catheters to pass to the left side of the heart. The event recognition module 8 may be configured to detect and highlight the time of a threshold pressure change, or reaching a threshold pressure, and to highlight when it was possible to cross between chambers. The recognized event is assigned an event type based on, for example, the pressure sensing modality and/or the time in the procedure where the threshold pressure change occurred. The pressure event type is associated with certain parameter values within the system-e.g., ultrasound, x-ray, iECG, etc. Thereby, the system 1 utilizes domain knowledge to assist in navigating through the procedure to locate and automatically display data associated with a particular selected event.

Each threshold triggered event detected by the event recognition module 8 may further include an event time and an event type. For example, the event recognition module 8 may be configured to assign the event time based on a time that the relevant threshold was exceeded, such as according to the reference clock 28. The event recognition module 8 may further be configured to determine the event type based on the modality and relevant threshold that was exceeded. For example, the event recognition module 8 may be configured to recognize an event when one or more temperature measurements from a temperature sensor on a catheter exceed a temperature threshold, when one or more pressure measurements from a pressure sensor on a catheter exceed a pressure threshold, and/or when one or more current measurements from a current sensor on a catheter exceed a current threshold. For instance, esophageal temperature monitoring data from an esophageal temperature probe may be analyzed during ablation, such as to assess whether the temperature data exceeds a relevant threshold or threshold change, and to detect an event accordingly. Marking such an event is important because it is possible to create a serious complication in ablation where excessive heating can cause lesions in the esophagus. Relevant parameters for the esophageal temperature event might include, for example, Thereby, temperature events related to esophageal temperature monitoring could be searched and selected, and the displayed datasets will automatically populate accordingly.

Alternatively or additionally, the event recognition module 8 may be configured to recognize and/or store other types of events. For example, the event recognition module 8 may be configured to recognize events, including determining an event time and an event type, based on user inputs to the system, such as user inputs via the user interface 30 and/or inputs to control one or more catheters 12 in the electrophysiology and vascular recording system 10. For example, the event recognition module 8 may be configured to recognize and document one or more procedure events marking occurrence of a step in the minimally invasive procedure, such as any patient preparation step, medication delivery step, catheter insertion step, ablation or stent placement, or the like. For example, a procedure event may be triggered based on a user input, such as a macro-input where a single input is associated with and initiates execution of multiple instructions. One of the instructions associated with the macro may be recognition of a particular procedure event by the event recognition module 8.

Similarly, the event recognition module may further be configured to identify one or more clinician marked events based on user input by a clinician via the one or more user input devices 32 to mark a particular time. Depending on the clinician input, the clinician marked event may mark a particular time in a particular modality data stream, or may mark a particular time in the overall procedure. Accordingly, the event recognition module 8 will determine an event type based on the form of the user input, such as whether the user input is provided to mark time in a single dataset or to mark a time in the procedure. Clinician marked events may be based on user input provided by a clinician to mark a dataset or an event in the procedure real-time as the event is occurring or the data is being collected, or the clinician input to generate a clinician marked event may be provided by the clinician while the clinician is reviewing previously-recorded datasets. For example, a clinician marked event may be a "bookmark" providing a time marker that can be utilized by a clinician during review to locate a particular point in the data. Such review commonly occurs during minimally invasive procedures, such as to make decisions on whether further studies or imaging are needed, or whether an intervention is warranted. Additionally, the user input to generate a clinician marked event may occur during post-procedure review, such as when a clinician is reviewing a procedure for documentation purposes.

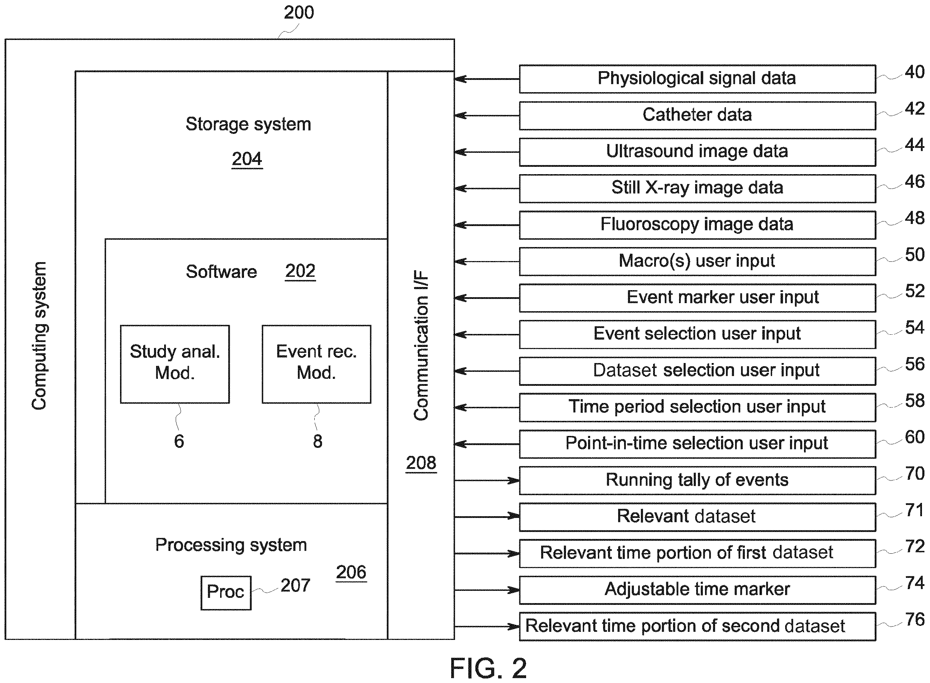

The study analysis module 6 and event recognition module 8 receive datasets, including physiological signal data, catheter data, image data, etc., and generate a time-synchronized and domain-aware user interface through which a clinician can review all datasets collected during a minimally invasive procedure, and can facilitate data analysis by identifying relevant datasets based on a number of factors, including events that have occurred in a procedure, datasets collected, a type of procedure, a current point in a procedure, user input by a clinician, etc. Figure 2 depicts a schematic diagram representing an exemplary embodiment of the computing system 200 comprising the study analysis module 6 and the event recognition module 8. The modules 6, 8 operate as described herein, utilizing modality dataset inputs and user inputs and generating various outputs to facilitate the analysis and review user interface and system.

In the example at Figure 2, modality dataset inputs include physiological signal data 40, such as may be provided by electrodes on one or more catheters 12, one or more surface leads 14, and/or any number of different physiological recording devices connectable to a patient to collect physiological signals. Dataset inputs also include catheter data 42 from physical modality sensors on the one or more catheters 12, such as pressure sensors, temperature sensors, and/or current sensors. The dataset inputs further include ultrasound image data from an ultrasound imaging device 20 providing ultrasound images of the patient's heart or vasculature. For example, the ultrasound image data 44 may be provided by an ultrasound imager on an endotracheal catheter. Image data may further include still x-ray image data 46, such as high-resolution x-rays taken by one or more x-ray systems within the catheterization laboratory or other procedure facility. Image data may further include fluoroscopy image data 48, which is a series of x-ray images taken in a short interval to capture movement. Fluoroscopy image data 48 is generally used to play a continuous series of x-ray images taken at a relatively fast frame rate such that movement (e.g., blood flow or movement of the heart muscle) can be captured and displayed, much like an x-ray movie. Accordingly, fluoroscopy image data 48 contains continuous series of images closely related to one another in time, and as such fluoroscopy image data generally utilizes a relatively large amount of memory space and processing power.

The computing system 200 is also configured to receive various user inputs. In the depicted example, the computing system receives macro user inputs 50, which as described above, are single instructions that expand automatically into a set of instructions to perform a set of tasks. Event marker user inputs 52 may also be provided, such as a clinician providing input via the user input devices 32 marking and event within a particular dataset or within the procedure as a whole, as is described above. Event selection user input 54 is also provided, which is user input to select one or more events from the running tally of events 70. Dataset selection user input 56 may also be received, which is user input to select a dataset or modality. The dataset selection user input 56 may be for selecting a dataset to be viewed within the analysis and review system 1, or to select a dataset for inclusion in the relevant datasets 71. A user may also provide time period selection user input 58 to select or adjust the time period of data displayed by the system 1. Additionally, the user may provide a point-in-time selection user input 60 to mark or select data at a particular point-in-time. For example, a point-in-time selection user input 60 may be utilized to select a particular point-in-time within one dataset that will be reflected in the display of the other relevant dataset data sets. To provide just one illustrative example, the user may provide a point-in-time selection user input 60 to select a point within the catheter data 42, which may cause display of corresponding image data captured at the selected point-in-time. Conversely, the user may provide a point-in-time selection user input 60 to select a point-in-time in the image data 44, 46, 48, which will cause the display to identify corresponding physiological signal data 40 and/or catheter data 42 to be visually identified on the display, depending on the current relevant datasets 71.

As described above, the event recognition module 8 recognizes events based on user input (e.g., event marker user input 52) and/or based on the data itself, and each recognized event gets added to the running tally of events 70, which is one output of the system. The study analysis module 6 generates and facilitates displaying of the data via the user interface 30. For example, the study analysis module 6 identifies relevant datasets 71 for display on one or more display devices 34 comprising part of the user interface 30. The study analysis module 6 further identifies a relevant time portion of each relevant dataset, and displays the relevant time portions of each relevant dataset 71 in a time-coordinated fashion so that the relevant time periods of the displayed data correspond and represent the same time period across all displayed datasets. As a user navigates through one displayed dataset, all other displays of data will update accordingly. Thus, if a user changes the period of time displayed for one dataset, the displays of all other datasets will be updated to display that same period of time. Thus, all datasets are displayed in a time-correlated, or time-locked, fashion so that the same time period is displayed across all of the review panes showing the various relevant datasets.

The study analysis module 6 may be configured to identify the relevant datasets 71 based on user input, such as datasets selected by a user. For example, the study analysis module 6 may identify relevant datasets 71 based on the datasets that a user is currently viewing. Alternatively or additionally, the study analysis module 6 may prompt or allow a user to provide input to select relevant datasets. In still other embodiments, the study analysis module 6 may automatically determine one or more relevant datasets 71 to be displayed, which may not be directly selected by a user, but are still based on certain user input. For example, a user may select an event or set of events within the running tally of events 70 (e.g., event selection user input 54), and the study analysis module 6 may determine the relevant datasets 71 based on the selected event, such as based on the event type of the selected event. For example, the study analysis module 6 may have a look-up table or other association table associating each of the various possible event types with a set of relevant parameter values, which are then used to identify the relevant datasets 71. To provide just one example, the event type may be associated with parameter values indicating a list of relevant data recording modalities-e.g., pressure sensor, ultrasound, and iECG-and the relevant datasets whose parameter values match those recording modalities are then identified.

Alternatively or additionally, the study analysis module 6 may select a set of relevant datasets 71 based on a selected portion of a particular dataset. For example, if a clinician views a portion of a particular dataset, the study analysis module 6 may be configured to select a set of relevant datasets 71 for display in conjunction with the selected portion of the dataset being viewed (e.g., by identifying key events occurring in the selected time period that relate to the dataset being viewed). For example, such an action by the study analysis module 6 may be triggered upon user input to engage the time-correlated analysis and review mode.

Once the relevant datasets are identified, the study analysis module 6 automatically displays those relevant datasets and provides a relevant time portion of each displayed relevant dataset 71. Figure 2 illustrates one exemplary output including a relevant time portion 71 of a first dataset and a relevant time portion 76 of a second dataset. The relevant time portions 72, 76 each represent the same period of time, albeit for two different types of data (e.g., recorded via different modalities). Additionally, the study analysis module 6 may generate one or more adjustable time markers 74 which may be displayed in conjunction with one or more of the relevant time portions 72, 76 and may allow a user to control the time portion of data being displayed across all of the datasets. For example, the adjustable time marker 74 may mark a particular point-in-time, such as a cursor or other marker that can be moved to isolate a particular point in a dataset. Alternatively, the adjustable time marker 74 may be a marker adjustable to designate a time window, such as calipers or a set of start and end markers to designate a period of time within a dataset.

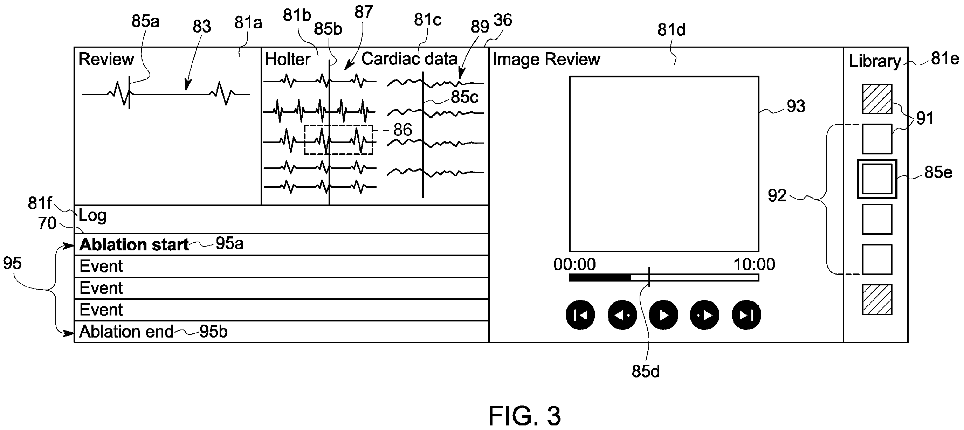

The relevant time portions 72, 76 may be determined based on the selected event or set of events, such as by identifying an event window that encapsulates a selected event or a selected point-in-time inputted by a user (e.g. via user inputs 54, 56, 58, or 60). The event window may be, for example, determined as a predetermined period of time on either side of a selected event time or a selected point-in-time. Alternatively, the event window may be based on a start time and end time of a selected event or set of events, as illustrated and described below regarding the example at Figure 3.

Figure 3 depicts an exemplary graphical user interface 36 provided on one or more display devices 34. The graphical user interface 36 is displaying multiple relevant datasets, including ECG from a holter monitor presented in review pane 81b, cardiac data presented in review pane 81c, and image data presented in review panes 81d and 81e. Additionally, review pane 81f is presented displaying the running tally of events 70. In the depicted example, a set of events 95 have been selected by a user. The set of datasets associated with an ablation event may be automatically selected and displayed upon receipt of the user input selecting the ablation event, or set of events. For example, the displayed set of relevant datasets in Figure 3 may be automatically selected based on the event type of the set of selected events 95, which is an ablation event including an ablation start and an ablation stop. Alternatively, the relevant datasets may be identified by the system accordingly to the datasets already being displayed on the graphical user interface 36. In still other embodiments, the relevant datasets may be identified based on user input, such as user input associating particular parameter values together or associating particular parameter values to one or more event types.

The selected events 95 include a starting event 95a and an ending event 95b at each of the start time and end time of an ablation portion of a procedure. Between the ablation start 95a and the ablation end 95b, three intervening events are marked. For example, the intervening events may be clinician marked events, procedure events, or threshold triggered events. The review panes 81b-81e provide physiological data, catheter data, and image data occurring during the selected set of events 95 for the relevant datasets. In the depicted embodiment, the relevant datasets are identified based on the selected events 95 (i.e., the ablation events), and are not identified based on the intervening events (which could be related or unrelated to the ablation events). In certain embodiments, the study analysis module 6 may be configured to identify whether any of the intervening events are related to the selected events 95, and if any of the intervening events are related then to further identify the relevant datasets based on the relevant intervening events as well.

In the example at Figure 3, review pane 81e provides ECG data 87 occurring between the event time of the ablation start event 95a and the event time of the ablation end event 95c. Review pane 81a displays the data in the region selected by the time focus window 86, which is another adjustable time marker that may be movable by a clinician in order to review the various datasets Review pane 81c provides multiple different catheter datasets 89. For example, the catheter data modalities may include temperature, pressure, current, and impedance measured by corresponding physical modality sensors on one or more invasive catheters inserted in the patient. Review pane 81d provides image data, which may include x-ray type image data and/or ultrasound image data. Thumbnails 91 of images are provided in review pane 81e. For example, the thumbnails 91 may include representative images of the captured image data. For example, where fluoroscopy image data is included in the available image data represented in review pane 81e, one still from each fluoroscopy image series may be represented as a thumbnail. If the series extends over a long period of time, multiple thumbnails may be presented in the image review pane 81e, each representing a period of fluoroscopy images.

In the depicted example, a set of representative images 92 between the ablation start and end times are highlighted to designate the relevant time portion of image data. Additionally, an identified image 93 is presented. The identified image 93 corresponds with the adjustable time marker 85 presented across all of the review panes identifying a particular point-in-time within the relevant time portion of a dataset. The identified image 93 is an image occurring at the selected point-in-time. In certain embodiments, each of the adjustable time markers 85a-85e can be moved by a user in order to adjust the time portions of the dataset displayed. Moving any one of the adjustable time markers 85a-85e will cause the markers in the remaining review panes to also be adjusted accordingly. Likewise, the adjustable time marker 85d in the image review pane 81d may be adjusted by playing the identified image 93, and the adjustable time markers 85b and 85c will move correspondingly to designate the point-in-time in the respective dataset that correlates with the image, or video frame, being played in the identified image window 93.

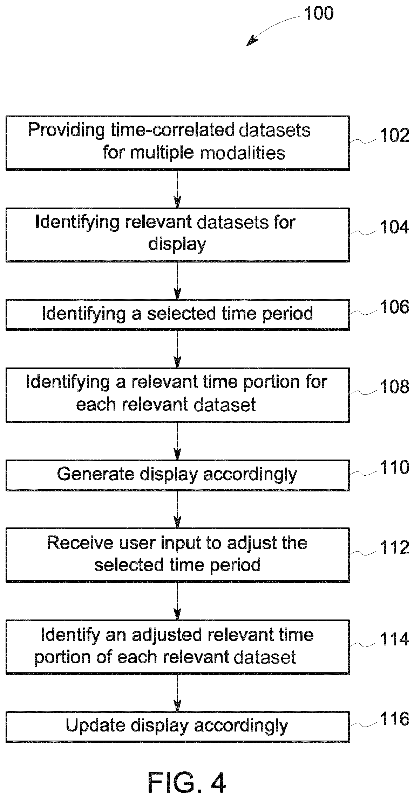

Figures 4-6 depict exemplary embodiments of methods 100, or portions thereof, of operating a computing system to facilitate analysis and review of datasets collected during a minimally invasive procedure. Time-correlated datasets for multiple modalities are provided at step 102. Relevant datasets are identified at step 104, such as based on user input selecting an event or based on datasets already being displayed to a user. A selected time period is identified at step 106, which may be based on user input selecting one or more events. Alternatively, the selected time period may be based on user input selecting a period for review within a particular dataset. All other datasets are then updated accordingly to provide the same selected time period worth of data. To that end, a relevant time portion for each relevant dataset is identified at step 108 and a display is generated accordingly at step 110 to display all of the relevant time portions of each relevant dataset. User input is received at step 112 to adjust the selected time period. For example, the user may adjust the selected time period by moving the adjustable time marker 85 in one of the review panes 81, or may adjust the selected time period by selecting a new event. An adjusted relevant time portion of each relevant dataset is identified at step 114, and the display is updated accordingly at step 116 to display the adjusted relevant time portions.

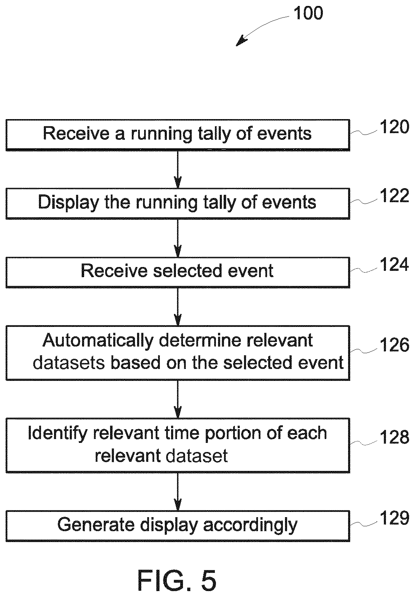

Figure 5 depicts another embodiment of a method of facilitating analysis and review of datasets collected during a minimally invasive procedure. A running tally of events is received at step 120, and the running tally of events is displayed at step 122 in a way that one or more of the events is selectable by a user. A selected event is received at step 124. One or more relevant datasets are automatically determined at step 126 based on the selected event, such as based on the event type of the selected event. Step 128 is then executed to identify the relevant time portion of each relevant dataset. The user interface display is then generated accordingly at step 129 to display the relevant time portions of each of the relevant datasets.

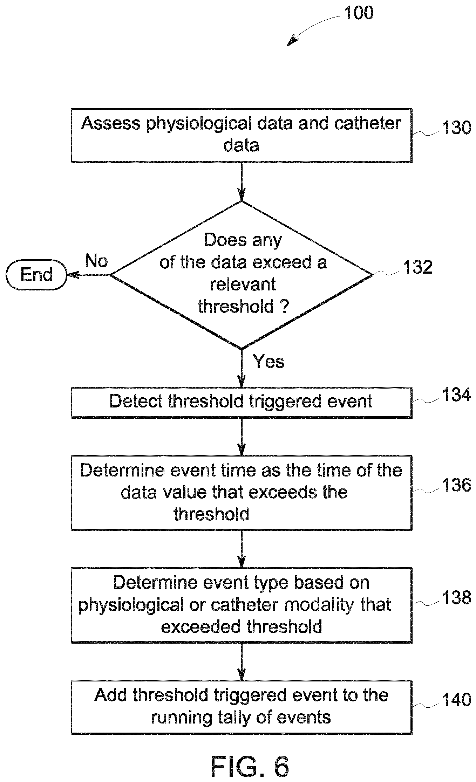

Figure 6 depicts a portion of the method showing event selection operation, such as may be executed by the event recognition module 8. The physiological data and catheter data, each subsets of the overall time-correlated multiple datasets, are assessed at step 130. If, at step 132, it is determined that any of the physiological data values or catheter data values exceed a relevant threshold, then a threshold trigger event is detected at step 134. For example, steps may be executed to determine each physiological signal dataset to determine whether it exceeds a relevant physiological threshold set for the respective physiological modality. Likewise, each catheter data set may be analyzed to determine whether any value therein exceeds a relevant measurement threshold set for a respective catheter modality. As used in reference to the threshold, the term "exceed" is meant to refer to a value that is above a high threshold or below a low threshold, depending on the relevant dataset and threshold. For example, thresholds for certain physiological modalities, such as blood pressure, SpO₂, heart rate, or the like, may include low thresholds. As used herein, "exceeding the threshold" includes data values that are below the relevant low thresholds set for the respective physiological modality, and also any values that are greater than a high threshold for the relevant dataset.

Once a threshold triggered event is detected at step 134, the event time and event type are recorded. The event time is determined at step 136 based on the time of the data value that exceeded the relevant threshold. For example, the event time may be identified as the time of the data value according to the reference clock. In certain embodiments, that may be local time stamp associated with the data value plus any offset for correlating the local clock to the reference clock. The event type is then determined at step 138 based on the physiological modality or catheter modality that exceeded the threshold. Alternatively or additionally, the event type may also be determined based on the relevant threshold that was exceeded, such as whether a low threshold or a high threshold for the relevant dataset was exceeded. Depending on the type of data (e.g., the modality represented by the data), exceeding a low threshold may be assigned to a different event type than exceeding a high threshold. The threshold triggered event is than added to the running tally of events at step 140.

Referring again to Figure 2, the computing system 200 includes a processing system 206, storage system 204, software 202, and communication interface 208. The processing system 206 loads and executes software 202 from the storage system 204, including study analysis module 6 and the event recognition module 8, which are applications within the software 202. Each of the modules 6,8 includes computer-readable instructions that, when executed by the computing system 200 (including the processing system 206), direct the processing system 206 to operate as described in herein in further detail, including to execute the steps to generate the graphical user interface providing time-synchronized and domain-aware review of all datasets collected during a minimally invasive procedure.

Although the computing system 200 as depicted in Figure 2 includes one software 202 encapsulating one study analysis module 6 and one event recognition module 8, it should be understood that one or more software elements having one or more modules may provide the same operation. Similarly, while description as provided herein refers to a computing system 200 and a processing system 206, it is to be recognized that implementations of such systems can be performed using one or more processors, which may be communicatively connected, and such implementations are considered to be within the scope of the description.

The processing system 206 includes the processor 207, which may be a microprocessor, a general purpose central processing unit, an application-specific processor, a microcontroller, or any other type of logic-based device. The processing system 206 may also include circuitry that retrieves and executes software 202 from storage system 204. Processing system 206 can be implemented within a single processing device but can also be distributed across multiple processing devices or sub-systems that cooperate in executing program instructions.

The storage system 204 can comprise any storage media, or group of storage media, readable by processing system 206, and capable of storing software 202. The storage system 204 can include volatile and non-volatile, removable and non-removable media implemented in any method or technology for storage of information, such as computer-readable instructions, data structures, program modules, or other data. Storage system 204 can be implemented as a single storage device but may also be implemented across multiple storage devices or sub-systems. Examples of storage media include random access memory, read only memory, optical discs, flash memory, virtual memory, and non-virtual memory, magnetic sets, magnetic tape, magnetic disc storage or other magnetic storage devices, or any other medium which can be used to store the desired information and that may be accessed by an instruction execution system, as well as any combination or variation thereof, or any other type of storage medium. Likewise, the storage media may be housed locally with the processing system 206, or may be distributed in one or more servers, which may be at multiple locations and networked, such as in cloud computing applications and systems. In some implementations, the storage media can be a non-transitory storage media. In some implementations, at least a portion of the storage media may be transitory.

The communication interface 208 interfaces between the elements within the computing system 200 and external devices, such as the user input device 32 and the display device 34 of the user interface 30. Additionally, the communication interface 208 may interface with the DICOM server 24 and/or the electrophysiology and vascular recording system 10 or imaging devices 20, 22.

The user interface 30 is configured to receive input from a user, such as a clinician, via one or more user input devices 32 and to facilitate provision of the graphical user interface 36. User input devices 32 may include a mouse, a keyboard, a voice input device, a touch input device for receiving a gesture from a user, a motion input device for detecting non-touch gestures and other motions by a user, and other comparable input devices and associated processing elements capable of receiving input from a user, such as a clinician. The user interface 30 further includes a display device 34, such as a video display or graphical display that can display a graphical interface as disclosed herein. Speakers, printers, haptic devices and other types of output devices may also be included in the user interface 210.

This written description uses examples to disclose the invention, including the best mode, and also to enable any person skilled in the art to make and use the invention. Certain terms have been used for brevity, clarity and understanding. No unnecessary limitations are to be inferred therefrom beyond the requirement of the prior art because such terms are used for descriptive purposes only and are intended to be broadly construed. The patentable scope of the invention is defined by the claims, and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A minimally invasive procedure analysis and review system comprising:
a display device (34);
a user input device (32);
a computing system communicatively connected to the display device 34 and the user input device 32;
a study analysis module (6) executable on a processor (207) and configured to:
receive a running tally of events (70) during the minimally invasive procedure, wherein each event includes an event time and an event type;
receive a selected event (72, 74) from the running tally of events (70);
determine at least two relevant datasets based on the event type of the selected event (72, 74), each dataset comprising one or more parameter (71) values, wherein the relevance determination is made based on the one or more parameter (71) values;
identify a relevant time portion of each relevant dataset based on the event time of the selected event (72, 74); and
display the relevant time portions (72, 74) of each of the at least two relevant datasets on the display device (34).

2. The system of claim 1, wherein the study analysis module (6) is further configured to receive multiple datasets, one for each of multiple modalities collected during the minimally invasive procedure, wherein the multiple datasets includes data from an electrophysiology and vascular recording system and image data from one or more imaging devices;
wherein all of the multiple datasets are time synchronized to a reference clock (28) and the event time for each event in the running tally of events (70) identified according to the reference clock 28; and
wherein the relevant time portion of each relevant dataset is identified according to the reference clock (28).

3. The system of claim (2), wherein the selected event (72, 74) includes a start time and an end time according to the reference clock (28), and wherein the relevant time portion includes data of each relevant dataset recorded between the start time and the end time.

4. The system of claim 3, wherein the study analysis module (6) is further configured to:
receive a user input to select a point-in-time within the start time and the end time of the selected event (72, 74); and
adjust the display for each of the at least two relevant datasets based on the selected point-in-time.

5. The system of claim 4, wherein at least one of the at least two relevant datasets includes a series of x-ray images, fluoroscopy images, or ultrasound images (92);
wherein the study analysis module (6) is configured to identify and display thumbnails (91) of representative x-ray images, fluoroscopy images, or ultrasound images (92) recorded between the start time and end time; and
wherein the study analysis module (6) is configured to, upon receipt of the selected point-in-time, identify an x-ray image, fluoroscopy image, or ultrasound image recorded at the point-in-time and adjust the display by displaying the identified image larger than the thumbnails (91).

6. The system of claim 1, wherein the relevant datasets include one or more of patient medical history and a lab result, and wherein the study analysis module (6) is further configured to:
identify one or more relevant portions of the patient medical history or the lab result based on the event type and/or the event time;
display the relevant portions of the patient medical or the lab result on the display device (34).

7. The system of claim 1, further comprising identifying an event window based on the event time and event type of the selected event (72, 74); and
wherein the relevant time portion of each relevant dataset is identified to correspond in time to the event window.

8. The system of claim 1, wherein the study analysis module (6) is further configured to receive multiple datasets, one dataset for each of multiple modalities collected during the minimally invasive procedure;
wherein the multiple datasets includes physiological signal data (40) from at least one physiological sensor and catheter data (42) from at least one physical modality sensor on a catheter;
further comprising an event recognition module (8) is executable on the processor (207) and configured to:
assess the physiological signal data (40) and the catheter data (42);
identify a threshold triggered event when the physiological signal data (40) exceeds a relevant physiological threshold set for a respective physiological modality or when the catheter data (42) exceeds a relevant measurement threshold set for a respective catheter modality, wherein the event time for the threshold triggered event is designated based on a time that the relevant physiological threshold or the relevant measurement threshold was exceeded and the event type for the threshold triggered event is designated based on the relevant physiological threshold or relevant measurement threshold that was exceeded; and
add the threshold triggered event to the running tally of events (70).

9. A method of operating a computing system to facilitate analysis and review of multiple datasets collected by an electrophysiology and vascular recording system and/or an imaging device during a minimally invasive procedure on a patient, wherein each dataset comprises one or more parameter (71) values, the computing system providing a graphical user interface on a display device (34) and receiving input from a user input device (32), the method comprising:
receiving a running tally of events (70) during a minimally invasive procedure, wherein each event includes an event time and an event type;
receiving a selected event (72, 74) from the running tally of events (70);
determining at least two relevant datasets out of the multiple datasets based on the event type of the selected event (72, 74), wherein the relevance determination is made based on the one or more parameter (71) values;
identifying a relevant time portion of each relevant dataset based on the event time of the selected event (72, 74); and
displaying the relevant time portions (72, 74) of each of the at least two relevant datasets on a display device (34).

10. The method of claim (9), further comprising receiving multiple datasets, one dataset for each of multiple modalities collected during the minimally invasive procedure, wherein the multiple datasets includes data from an electrophysiology and vascular recording system and image data from one or more imaging devices;
wherein all of the multiple datasets are time synchronized to a reference clock (28) and the event time for each event in the running tally of events (70) identified according to the reference clock (28); and
wherein the relevant time portion of each relevant dataset is identified according to the reference clock (28).

11. The method claim 10, wherein the selected event (72, 74) includes a start time and an end time according to the reference clock (28), and wherein identifying the relevant time portion includes identifying the data of each relevant dataset recorded between the start time and the end time.

12. The method of claim (11), further comprising:
receiving a user input to select a point-in-time within the start time and the end time of the selected event (72, 74); and
adjusting the display for each of the at least two relevant datasets based on the selected point-in-time.

13. The method of claim (12), wherein at least one of the at least two relevant datasets includes x-ray images or fluoroscopy images and further comprising:
identifying and displaying thumbnails (91) of the x-ray images or fluoroscopy images recorded between the start time and end time; and
upon receipt of the selected point-in-time, identifying an x-ray image or fluoroscopy image recorded at the point-in-time and adjusting the display by displaying the identified image larger than the thumbnails (91).

14. The method of claim (9), wherein the multiple datasets collected by the electrophysiology and vascular recording system includes physiological signal data 40 from at least one physiological sensor and catheter data (42) from at least one physical modality sensor on a catheter, and further comprising:
identifying a threshold triggered event when the physiological signal data (40) exceeds a relevant physiological threshold set for a respective physiological modality or when the catheter data (42) exceeds a relevant measurement threshold set for a respective catheter modality, wherein the event time for the threshold triggered event includes is designated based on a time that the relevant physiological threshold or the relevant measurement threshold was exceeded and the event type for the threshold triggered event is designated based on the relevant physiological threshold or relevant measurement threshold that was exceeded; and
add the threshold triggered event to the running tally of events (70).

15. A method of operating a computing system to facilitate analysis and review of data collected during a minimally invasive procedure on a patient, the computing system providing a graphical user interface on a display device (34) and receiving input from a user input device (32), the method comprising:
providing multiple datasets, one for each of multiple modalities collected during the minimally invasive procedure, wherein each dataset comprises one or more parameter (71) values and wherein all of the datasets include a time parameter (71) synchronized to a single reference clock (28);
identifying at least two relevant datasets for display out of the multiple datasets, wherein the relevance determination is made based on the one or more parameter (71) values of the relevant datasets;
identifying a selected time period according to the reference clock (28);
identifying a relevant time portion of each relevant dataset based on the selected time period;
displaying the relevant time portions (72, 74) of each of the at least two relevant datasets on a display device (34);
receiving a user input to adjust the selected time period;
identifying an adjusted selected time period based on the user input and according to the reference clock (28);
identifying an updated relevant time portion of each relevant dataset to include data occurring during the adjusted selected time period; and
adjusting the display for each of the at least two relevant datasets to display the updated relevant time portions (72, 74) of each of the at least two relevant datasets on the display device (34).
